# EUROPÄISCHE PATENTSCHRIFT

(11) **EP 1 182 979 B1**
(45) Veröffentlichungstag und Bekanntmachung des Hinweises auf die Patenterteilung: **17.08.2005**
(21) Anmeldenummer: 00940307.2
(22) Anmeldetag: 03.06.2000
(51) Int. Cl.: A61B 18/00, A61B 18/20

(54) **VORRICHTUNG ZUM ABSAUGEN VON ABPRODUKTEN BEI DER ABLATION VON BIOLOGISCHEM GEWEBE**
APPARATUS FOR REMOVAL OF WASTE PRODUCTS BY SUCTION DURING THE ABLATION OF BIOLOGICAL TISSUE
DISPOSITIF D'ASPIRATION DE DECHETS LORS DE L'ABLATION D'UN TISSU BIOLOGIQUE

(30) Priorität: 07.06.1999 DE 19927016; 25.04.2000 DE 10020522
(43) Veröffentlichungstag der Anmeldung: 06.03.2002
(73) Patentinhaber: Carl Zeiss Meditec AG, 07745 Jena (DE)
(72) Erfinder: FIEDLER, Joachim, D-74564 Crailsheim (DE); GODER, Claus, D-90491 Nürnberg (DE); SCHRÖDER, Eckhard, D-90542 Eckental (DE); SEITZ, Bernhard, D-07751 Jena (DE)
(74) Vertreter: Niestroy, Manfred
(86) Internationale Anmeldenummer: PCT/EP2000/005079
(87) Internationale Veröffentlichungsnummer: WO 2000/074581

(56) Entgegenhaltungen:
- DE-A- 19 710 676
- US-A- 5 181 916
- US-A- 5 344 418
- US-A- 5 626 568
- US-A- 5 630 807

## Beschreibung

### Gebiet der Erfindung

Die Erfindung bezieht sich auf eine Anordnung zur Absaugung von Abprodukten, wie Rauch und Gewebepartikel, bei der Ablation von biologischem Gewebe mit Hilfe von Laserstrahlung, wobei die Laserstrahlung durch die Mündungsöffnung eines röhrenförmigen Kanals hindurch auf das Gewebe gerichtet und die Abprodukte durch die Mündungsöffnung in den Kanal hinein abgesaugt werden.

### Stand der Technik

Es ist bekannt, biologisches Gewebe durch Einbringen von Laserenergie ohne wesentliche thermische Schädigung der Zielgebiete abzutragen. Dieser quasi nichtthermische Prozeß wird beispielsweise in der Medizin zur Bearbeitung von Knorpel, Zahnhartgewebe, Hautarealen und auch bei der Augenchirurgie zur Formung der Hornhaut genutzt (Photorefraktive Keratektomie). Derartige Verfahren und zugehörige Vorrichtung sind beispielsweise in DE 197 27 573 C1 und EP 0 412 789 B1 veröffentlicht.

Nachteilig ist, daß die bei der Abtragung des Gewebes entstehenden Abprodukte in Form von Rauch oder Gewebepartikeln die Luftqualität in unmittelbarer Nähe des Behandlungsortes beeinträchtigen, was einerseits zur Geruchsbelästigung für den Patienten und das behandelnde Personal führt, andererseits aber auch dazu, daß die Laserstrahlung partiell geschwächt wird. Letzteres ist insbesondere bei der photorefraktiven Keratektomie von Bedeutung, bei der die Hornhautoberfläche durch präzisen Materialabtrag geformt wird. Hier kommt es neben einer unbehinderten, feinfühligen Ausrichtbarkeit des Laserstrahles auch darauf an, daß die Strahlungsenergie bei exakt gleichbleibender Intensität in die Hornhaut eingebracht wird, damit das Ablationsergebnis in der gewünschten Qualität erzielt werden kann. Die Intensität aber wird durch abziehende und dabei den Laserstrahl durchquerende Rauchwolken und Gewebepartikel beeinflußt, was zu irregulären und somit unerwünschten Veränderungen der Ablationen führen kann.

In der US-Patentschrift 5,344,418 ist eine Anordnung beschrieben, bei der ein Applikator nahe der Austrittsöffnung für den Laserstrahl Strömungskanäle für Gase bzw. für Luft aufweist, aus denen während der Behandlung ein Gas- bzw. Luftstrom auf das behandelte Gewebe gerichtet sind, was wie beabsichtigt zur Folge hat, daß die störenden Ablations-Abprodukte vom Behandlungsareal weggeblasen werden.

Abgesehen davon, daß hiermit das Problem der Luftverunreinigung und der Geruchsbelästigung für Patient und Operateur nicht gelöst ist, macht sich weiterhin ein Nachteil insofern bemerkbar, als die Luftströmung, die sich dabei relativ zur Oberfläche der behandelten biologischen Substanz ergibt, zu einer störenden Austrocknung dieser Substanz führt. Auch diese Austrocknung verursacht nicht vorausberechenbare und häufig zu starke Ablationen.

Die genannten Nachteile vermag auch die mit der US-Patentschrift 5,181,916 dargelegte technische Lösung nicht zu beseitigen. Hier ist zwar kein Gasstrom in der Weise auf das behandelte Areal gerichtet, daß die Verunreinigungen weggeblasen werden, sondern die am Behandlungsort entstehenden Rauchgase werden mit Hilfe des Gasstromes abgesaugt. Hierzu dient eine Ansaugöffnung, die konzentrisch um die Mündungsöffnung, durch die der Laserstrahl austritt, angeordnet ist. Auch dabei entsteht aufgrund des über das Gewebe hinwegströmenden Gases eine Austrocknung des Gewebes.

Von diesem Stand der Technik ausgehend besteht die Aufgabe der Erfindung darin, unter Beibehaltung der Vorteile der Beseitigung der Ablations-Abprodukte durch Absaugung die durch die Ablations-Abprodukte verursachten unerwünschten Schwankungen der Laserstrahlungsintensität zu verringern und auch die Austrocknung der behandelten Substanz weitestgehend zu vermeiden.

Erfindungsgemäß ist bei einer Anordnung der eingangs genannten Art vorgesehen, ***daß die Kanalinnenwandung in der Nähe der Mündungsöffnung mindestens*** *eine Ausströmöffnung für einen Gasstrom aufweist, der zur Mitte des Kanals gerichtet ist oder im wesentlichen entgegengesetzt zur Abstrahlungsrichtung des Laserstrahles in den Kanal hinein gerichtet ist.*

In einer besonders bevorzugten Ausgestaltung der Erfindung weist der röhrenförmige Kanal einen sich zur Mündungsöffnung hin konisch verjüngenden Abschnitt auf.

Im Rahmen dieser Erfindung liegt es weiterhin, wenn der röhrenförmige Kanal zumindest im dem konisch verlaufenden Abschnitt zwei konzentrisch um seinen Umfang verlaufende Kammern aufweist, wovon eine Kammer zur Zuführung des Gases zu der Ausströmöffnung vorgesehen ist. Die zweite Kammer dient zur Abführung des abgesaugten Gases, welches dann die Abprodukte mitführt, aus dem Kanalinneren. Sie ist zu diesem Zweck einerseits über eine Absaugöffnung mit dem Kanalinneren und andererseits über eine Saugleitung mit einer Absaugeinrichtung verbunden.

Mit dieser Anordnung wird wirkungsvoll erreicht, daß das Gas nicht zwischen der Mündungsöffnung und dem Behandlungsareal hindurch nachströmt und dabei die Substanzoberfläche überstreicht, sondern bereits aus der Mündungsöffnung bzw. aus der unmittelbaren Nähe der Mündungsöffnung unter angemessenem Druck austritt und in das Kanalinnere hinein gelenkt wird.

Damit wird der durch die Absaugung entstehende Unterdruck überwiegend oder auch völlig mit dem durch die Ausströmöffnungen nachgeführten Gasvolumen ausgeglichen, was dazu führt, daß außerhalb des Kanals, insbesondere zwischen der Mündungsöffnung und der Substanzoberfläche, nur noch eine geringe bzw. keine Luftströmung mehr entsteht und so eine Austrocknung nicht mehr stattfinden kann.

Der bei der Behandlung entstehende und vom Behandlungsort kommende Rauch bzw. die sich von dort entfernenden Gewebepartikel bewegen sich in Richtung der Mündungsöffnung, werden dort von der Gas- bzw. Luftströmung innerhalb des röhrenförmigen Kanals erfaßt und abtransportiert.

In vorteilhaften Ausgestaltungen können beispielsweise radialsymmetrisch um die Kanalmitte angeordnete Ausströmöffnungen oder, alternativ hierzu, eine ringförmig umlaufende Ausströmöffnung vorgesehen sein. In beiden Varianten wird eine vergleichsmäßige Gasströmung erzielt, die zunächst von den Ausströmöffnungen in der Mündung zur Kanalmitte hin gerichtet ist und dann aufgrund des im Kanalinneren ausgebildeten Saugunterdruckes entgegengesetzt zur Richtung des Laserstrahles in den Kanal hinein umgelenkt wird.

Bevorzugt sind die Ausströmöffnungen diffusorartig aufgeweitet, so daß das Gas mit geringer werdender Strömungsgeschwindigkeit aus diesen Ausströmöffnungen austritt und so eine unerwünschte Verwirbelung des Gasstromes vermieden wird. Vor allem wird so verhindert, daß sich das aus den Ausströmöffnungen austretende Gas aufgrund von Verwirbelungen oder zu hoher Ausströmgeschwindigkeit von der Mündungsöffnung zum Behandlungsareal hin bewegt.

In einer besonders bevorzugten Ausgestaltung der Erfindung ist als Gas Luft vorgesehen und die Ausströmöffnungen sind mit einem Luftverdichter oder mit einem mit Luft gefüllten Druckbehälter verbunden. Vorteilhaft sollten an dem Luftverdichter bzw. an dem Druckbehälter Mittel zur Regulierung des Druckes der zugeführten Luft und damit auch der Strömungsgeschwindigkeit vorhanden sein. Derartige Mittel, wie beispielsweise Druckminderventile, sind im Stand der Technik hinreichend bekannt und müssen hier nicht näher beschrieben werden.

Weiterhin kann vorteilhaft vorgesehen sein, daß die Innenwandung des Kanals mehrere radialsymmetrisch um die Kanalmitte angeordnete Absaugöffnungen aufweist, die mit einer Absaugeinrichtung in Verbindung stehen. Mit dieser Anordnung wird auch eine Verwirbelung der von den Austrittsöffnungen austretenden und zu den Absaugöffnungen gerichteten, die Rauchgase bzw. Gewebepartikel mitführenden Luft verhindert, da zwischen Austrittsöffnung und Absaugöffnung immer nur der kürzeste Weg zurückzulegen ist.

Die Luftströmung findet dabei im wesentlichen nahe der Innenwandung des Kanals statt, während das Zentrum, das der Laserstrahlung vorbehalten ist, nur in geringem Maße von dem Luftstrom beeinträchtigt ist. Das führt auch dazu, daß sich Rauchgas und Gewebepartikel mit dem Luftstrom weitestgehend außerhalb der Laserstrahlung bewegen und dort abgeleitet werden, so daß die auf das Behandlungsareal auftreffende Energie bezüglich ihrer Intensität nicht oder nur in wesentlich geringerem Ausmaß als beim Stand der Technik von nichttransparenten Teilchen beeinträchtigt wird.

Bevorzugt ist weiterhin vorgesehen, daß der Gesamtquerschnitt der Ausströmöffnungen und der Gesamtquerschnitt der Absaugöffnungen, der Überdruck an den Ausströmöffnungen und der Unterdruck an den Absaugöffnungen wie auch die Strömungsgeschwindigkeiten in den Ausströmöffnungen und die Strömungsgeschwindigkeiten in den Absaugöffnungen so aufeinander abgestimmt sind, daß die je Zeiteinheit abgesaugte Luftmenge um einen Faktor, der zwischen 1,1 und 1,3 liegt, größer ist als die durch die Ausströmöffnungen zugeführte Luftmenge.

Auf diese Weise ist dafür gesorgt, daß die zugeführte Luftmenge etwas geringer ist als die abgesaugte Luftmenge und deshalb nicht vollständig ausreicht, um die abgesaugte Luftmenge druckausgleichend zu ersetzen, so daß auch noch eine der Differenz entsprechende Luftmenge von außerhalb durch die Mündungsöffnung hindurch in den Kanal gesaugt wird. Das führt zu einer geringfügigen Luftströmung nahe und außerhalb der Mündungsöffnung, die dazu dient, die von der Behandlungsstelle aufsteigenden Rauchgase bzw. Gewebepartikel zu erfassen und in den ringförmigen Kanal hineinzusaugen. Dieser Faktor und damit die von außerhalb des Kanals angesaugte Luftmenge kann durch Mittel reguliert werden, die zur Beeinflussung des Druckes der zugeführten Luft bzw. zur Beeinflussung der Strömungsgeschwindigkeit der zugeführten Luft vorgesehen sind.

Mit der erfindungsgemäßen Anordnung wird äußerst effektiv erreicht, daß die Geruchsbelästigung für den Patienten und den behandelnden Arzt aufgehoben ist, die Energiedichte der Laserstrahlung durch das Freihalten von Rauchgasen bzw. Gewebepartikeln über die Zeitdauer der Operation hinweg weitestgehend gleichmäßig bleibt und auch Luftströmungen vom Behandlungsareal weitestgehend ferngehalten werden.

In einer weiteren bevorzugten Ausgestaltung der Erfindung kann eine Einrichtung zum abwechselnden Unterbrechen der auf das Gewebe treffenden Laserstrahlung einerseits und der Luftströmung andererseits vorgesehen sein. Damit wird vorteilhaft erreicht, daß nach jeweils einer Behandlungsphase, in der die Laserstrahlung auf das Gewebe gerichtet war und eine Teilablation erfolgt ist, eine Absaugphase folgt, während der in der bereits beschriebenen Weise Luft zugeführt bzw. abgesaugt wird und dabei die Ablationsprodukte abtransportiert werden. Nach Ende dieser "Reinigungsphase" wird der Luftstrom unterbrochen und es schließt sich erneut eine Behandlungsphase an. Auch hierbei wird erreicht, daß die Ablationsprodukte abgesaugt werden, insofern keine Geruchsbelästigung entsteht und auch die Laserstrahlung frei von nichttransparenten Teilchen gehalten wird, so daß auf das Gewebe eine Strahlung mit gleichbleibender Energiedichte treffen kann.

In einer weiteren Ausgestaltung schließlich kann auch vorgesehen sein, daß der Kanal relativ zur Laserstrahlung verschwenkbar angeordnet ist, wobei in einer bevorzugten Ausrichtung des Kanals die Laserstrahlung vom Kanal umschlossen ist. So ist es dem behandelnden Arzt beispielsweise möglich, für Phasen der Behandlung, die eine bessere Sicht auf das Behandlungsareal erfordern, den röhrenförmigen Kanal zur Seite zu schwenken.

*Die Erfindung bezieht sich weiterhin auf ein Verfahren zum Betreiben der vorstehend beschriebenen Anordnung zur Absaugung von Abprodukten, wie Rauch und Gewebepartikel, bei einer Ablation mit Hilfe von Laserstrahlung, wobei die während der Abtragung entstehenden Abprodukte in einer Phase vom Behandlungsort entfernt werden, in der nicht gleichzeitig die Laserstrahlung chirurgisch aktiv eingesetzt ist.*

Vorteilhaft erfolgt die Entfernung der Abprodukte mit einem Gas- bzw. Luftstrom, der nicht auf das behandelte Gewebe gerichtet ist und diese auch nicht oder nur in geringem Maße überstreicht, um so eine Austrocknung des Gewebes zu vermeiden.

### Kurze Beschreibung der Zeichnungen

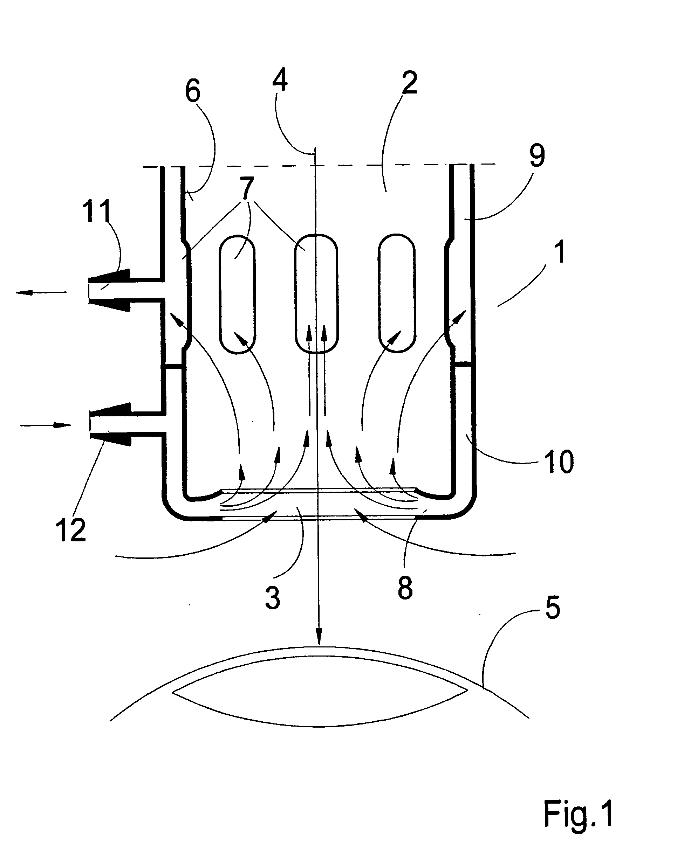

Die Erfindung soll nachfolgend anhand zweier Ausführungsbeispieles näher erläutert werden. Die zugehörigen Zeichnungen zeigen in
- Fig. 1: die Prinzipdarstellung der Erfindung in einem ersten Ausführungsbeispiel, bei dem die Mündungsöffnung eine Ausströmöffnung für ein Gas aufweist, wobei die Strömungsrichtung des Gases zur Kanalmitte gerichtet ist und in
- Fig.2: die Prinzipdarstellung der Erfindung anhand eines zweiten Ausführungsbeispieles, bei dem die Mündungsöffnung ebenfalls eine Ausströmöffnung für ein Gas aufweist, wobei jedoch die Strömungsrichtung des Gases im wesentlichen entgegengesetzt zur Abstrahlung des Laserstrahles in den Kanal hinein gerichtet ist.

### Ausführliche Beschreibung der Zeichnungen

Dargestellt ist das Mundstück 1 einer Einrichtung, die beispielhaft zur photorefraktiven Keratektomie dient, bei der die Krümmung der Hornhaut des Auges unter Einwirkung der Laserstrahlung gezielt und abschnittsweise durch Materialabtrag korrigiert wird. Das Mundstück 1 besteht im wesentlichen aus einem röhrenförmigen Kanal 2, durch dessen Mündungsöffnung 3 hindurch ein Laserstrahl 4 auf die Hornhaut 5 gerichtet ist.

Bei dieser Behandlung kommt es zur Bildung von Rauchgas und es gehen außerdem von der behandelten Stelle an der Hornhaut 5 auch ablatierte Gewebepartikel aus.

Um nun zu verhindern, daß sich Rauchgase und Gewebepartikel zeitweise in der Laserstrahlung aufhalten und als nicht transparente Teilchen die in die Hornhaut 5 einzustrahlende Energiedichte verringern, sind in der Innenwandung 6 des Kanals 2 mehrere Absaugöffnungen 7 radialsymmetrisch um die Kanalmitte, in der vorzugsweise der Laserstrahl 4 verläuft, angeordnet. Die Absaugöffnungen 7 stehen mit einer Absaugeinrichtung in Verbindung, die einen Unterdruck erzeugt, infolge dessen Luft durch die Absaugöffnungen 7 hindurch aus dem Kanalinneren abgesaugt wird und mit dieser abgesaugten Luft auch die Rauchgase bzw. die von der Hornhaut 5 abspringenden Gewebepartikel abtransportiert werden.

Um außerdem zu verhindern, daß die infolge des Druckausgleichs nachströmende Luft die zu behandelnde Stelle der Hornhaut 5 ausgetrocknet und um zugleich auch zu vermeiden, daß die Rauchgase und die abspringenden Gewebepartikel sich überhaupt oder auch nur zu lange im Laserstrahl 4 aufhalten, ist erfindungsgemäß vorgesehen, daß in der Mündungsöffnung 3 eine Ausströmöffnung 8 für Luft vorgesehen ist.

Die Ausströmöffnung 8 ist ringförmig umlaufend ausgebildet und zentrisch zur Kanalmitte angeordnet. Die Ausströmöffnung 8 ist zur Kanalmitte gerichtet und so geformt, daß sich ihr Querschnitt in Strömungsrichtung erweitert, so daß sich die Strömungsgeschwindigkeit der zur Kanalmitte hin strömenden Luft verringert.

In der Zeichnung ist weiterhin zu erkennen, daß das Mundstück 1 als doppelwandiges Rohr mit voneinander getrennten Kammern 9 und 10 ausgebildet ist. Die Kammer 9 steht mit einem Anschlußstutzen 1 1 und die Kammer 10 mit einem Anschlußstutzen 12 in Verbindung. Die Anschlußstutzen 11, 12 sind beispielsweise als Schlauchanschlüsse ausgebildet und können so über Schläuche mit einer Absaugvorrichtung (im Beispiel die Kammer 9 und den Anschlußstutzen 11 betreffend) bzw. mit einem Verdichter oder einem Druckluftbehälter (im Beispiel die Kammer 10 und den Anschlußstutzen 12 betreffend) verbunden sein.

Mit entsprechenden Mitteln zur Regulierung von Luftströmen, die an und für sich bekannt sind, können die durch den Anschlußstutzen 12 zugeführte Luftmenge und die durch den Anschlußstutzen 11 abgeführte Luftmenge so aufeinander abgestimmt sein, daß die zugeführte Luftmenge ausreicht, um den mit der abgeführten Luftmenge erzeugten Unterdruck auszugleichen, wodurch verhindert wird, daß im Bereich zwischen der Mündungsöffnung 3 und der Oberfläche der Hornhaut 5 eine Luftströmung entsteht, durch die im Stand der Technik der Saugunterdruck ausgeglichen wird.

Damit wird erreicht, daß im Bereich zwischen der Mündungsöffnung 3 und der Oberfläche der Hornhaut 5 keine bzw. nur äußerst geringe Luftbewegungen stattfinden, so daß die Austrocknung der Oberfläche der Hornhaut 5 vermieden wird.

Im Rahmen der Erfindung liegt es auch, daß die zugeführte und die abgesaugte Luftmenge so aufeinander abgestimmt sind, daß die zugeführte Luftmenge nicht vollständig ausreicht, um den Saugunterdruck auszugleichen, so daß durch die Mündungsöffnung 3 von außen her noch Luft nachströmen muß, ohne daß dabei jedoch in unmittelbarer Nähe der Oberfläche der Hornhaut 5 zu starke, die Substanz austrocknende Luftströmungen oder Luftverwirbelungen entstehen. Die von außen her nachströmende Luftmenge ist zwar geringfügig, unterstützt aber das Absaugen der bei der Ablation entstehende Rauchgase und abspringenden Gewebepartikel.

Die vorgenannten Effekte und Wirkungen werden beispielsweise erzielt, wenn die Mündungsöffnung 3 einen Durchmesser von etwa 40mm hat und der Abstand zwischen Mündungsöffnung 3 und Oberfläche des Behandlungsareals etwa 50 mm beträgt. Aus der Ausströmöffnung 8 sollte etwa ein Luftvolumen von 40 Liter pro Minute ausströmen. Die abgesaugte Luftmenge sollte in derselben Zeiteinheit zwischen 40 und 55 Liter betragen.

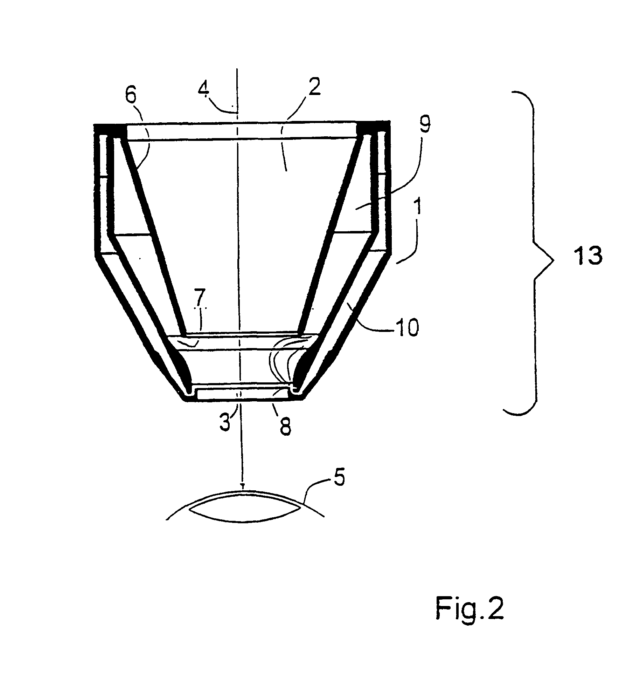

In Fig.2 ist eine weiteres Ausführungsbeispiel einer erfindungsgemäßen Anordnung zur Absaugung von Abprodukten bei der Ablation von biologischem Gewebe dargestellt.

Hier weist der röhrenförmige Kanal 2 ebenfalls eine Mündungsöffnung 3 auf, in deren Nähe eine Ausströmöffnung 8 vorgesehen ist. Die Besonderheit hierbei besteht allerdings darin, daß die Strömungsrichtung des Gases von der Ausströmöffnung 8 im wesentlichen entgegengesetzt zur Abstrahlungsrichtung des Laserstrahles 4 in den Kanal 2 hinein gerichtet ist. Auf diese Weise wird wirkungsvoll erreicht, daß das Gas nicht durch den Freiraum zwischen der Mündungsöffnung 3 und der Hornhaut 5 hindurch nachströmt und dabei die Hornhautoberfläche überstreicht und diese austrocknet, sondern bereits mit dem Austritt aus Ausströmöffnung 8 in das Kanalinnere hinein gelenkt wird. Dabei sind die Druckverhältnisse so abgestimmt wie weiter oben bereits beschrieben.

Hiermit wird auf besonders vorteilhafte Weise erreicht, daß die Ablationsprodukte abgesaugt werden, ohne daß dabei die Intensität der Laserstrahlung wesentlich beeinträchtigt wird.

In Fig.2 ist weiterhin zu erkennen, daß der röhrenförmige Kanal 2 in diesem Falle einen konisch verlaufenden Abschnitt (13) aufweist. Auch hier ist, wie schon im weiter oben beschriebenen Ausführungsbeispiel, das Mundstück 1 mit voneinander getrennten Kammern 9 und 10 versehen, die konzentrisch um den Umfang verlaufen. Davon ist eine Kammer (10) zur Zuführung des Gases zu der Ausströmöffnung (8) und die zweite Kammer (9) zur Abführung des abgesaugten Gases einschließlich der Abprodukte vorgesehen.

Zu diesem Zweck stehen die Kammern 9, 10 jeweils mit Anschlußstutzen (hier nicht dargestellt) in Verbindung, die beispielsweise als Schlauchanschlüsse ausgebildet und über Schläuche mit einer Absaugvorrichtung (die Kammer 9 betreffend) bzw. mit einem Verdichter oder einem Druckluftbehälter (die Kammer 10 betreffend) verbunden sind.

## Patentansprüche

1. Anordnung zur Absaugung von Abprodukten, wie Rauch und Gewebepartikel, bei der Ablation von biologischem Gewebe mit Hilfe von Laserstrahlung, wobei die Laserstrahlung durch die Mündungsöffnung (3) eines röhrenförmigen Kanals (2) hindurch auf das Gewebe gerichtet und die Abprodukte durch die Mündungsöffnung (3) in den Kanal (2) hinein abgesaugt werden, **dadurch gekennzeichnet, daß** die ***Kanalinnenwandung*** in der Nähe der Mündungsöffnung (3) mindestens eine ***Ausströmöffnung*** **(8)** für einen Gasstrom aufweist, der zur Mitte des Kanals (2) gerichtet ist oder im wesentlichen entgegengesetzt zur Abstrahlungsrichtung des Laserstrahles (4) in den Kanal (2) hinein gerichtet ist.

2. Anordnung nach Anspruch 1, **dadurch gekennzeichnet, daß** der röhrenförmige Kanal (2) einen sich zur Mündungsöffnung (3) hin konisch verjüngenden Abschnitt (13) aufweist.

3. Anordnung nach Anspruch 2, **dadurch gekennzeichnet, daß** der röhrenförmige Kanal (2) zumindest in dem konisch verlaufenden Abschnitt (13) zwei konzentrisch um seinen Umfang verlaufende Kammern (9,10) aufweist, wovon eine Kammer (10) zur Zuführung des Gases zu der Ausströmöffnung (8) und die zweite Kammer (9) zur Abführung des abgesaugten Gases einschließlich der Abprodukte vorgesehen ist.

4. Anordnung nach einem der vorgenannten Ansprüche, **dadurch gekennzeichnet, daß** mehrere Ausströmöffnungen (8) vorgesehen und radialsymmetrisch um die Kanalmitte angeordnet sind.

5. Anordnung nach einem der Ansprüche 1-3, **dadurch gekennzeichnet, daß** eine ringförmig umlaufende Ausströmöffnung (8) vorgesehen ist.

6. Anordnung nach einem der vorgenannten Ansprüche, **dadurch gekennzeichnet, daß** die Ausströmöffnungen (8) diffusorartig aufgeweitet sind.

7. Anordnung nach einem der vorgenannten Ansprüche, **dadurch gekennzeichnet, daß** als Gas Luft vorgesehen ist und die Ausströmöffnungen (8) mit einem Kompressor oder einem mit Luft gefüllten Druckbehälter verbunden sind.

8. Anordnung nach einem der vorgenannten Ansprüche, **dadurch gekennzeichnet, daß** die Innenwandung (6) des Kanals (2) mehrere radialsymmetrisch um die Kanalmitte angeordnete Absaugöffnungen (7) aufweist, die mit einer Absaugeinrichtung in Verbindung stehen.

9. Anordnung nach einem der vorgenannten Ansprüche, **dadurch gekennzeichnet, daß**
- der Gesamtquerschnitt der Ausströmöffnungen (8) und der Gesamtquerschnitt der Absaugöffnungen (7),
- der Überdruck an den Ausströmöffnungen (8) und der Unterdruck an den Absaugöffnungen (7) und
- die Strömungsgeschwindigkeiten in den Ausströmöffnungen (8) und die Strömungsgeschwindigkeiten in den Absaugöffnungen (7)
so aufeinander abgestimmt sind, daß die je Zeiteinheit abgesaugte Luftmenge um den Faktor 1,1 bis 1,3 größer ist als die durch die Ausströmöffnungen (8) zugeführte Luftmenge.

10. Anordnung nach einem der vorgenannten Ansprüche, **dadurch gekennzeichnet, daß** eine Einrichtung zum abwechselnden Unterbrechen der auf das Gewebe treffenden Laserstrahlung und der Zuführung und Absaugung von Luft vorgesehen ist.

11. Anordnung nach einem der vorgenannten Ansprüche, **dadurch gekennzeichnet, daß** der Kanal relativ zur Laserstrahlung verschwenkbar angeordnet ist, wobei in einer bevorzugten Ausrichtung des Kanals (2) die Laserstrahlung vom Kanal (2) umschlossen ist.

12. Verfahren zum Betreiben einer Anordnung nach den Ansprüchen 1 bis 1 zur Absaugung von Abprodukten, wie Rauch und Gewebepartikel, bei einer Ablation mit Hilfe von Laserstrahlung, wobei die während der Abtragung entstehenden Abprodukte ***in einer Phase*** vom Behandlungsort entfernt werden, *in der nicht gleichzeitig die Laserstrahlung chirurgisch aktiv eingesetzt ist*.

13. Verfahren nach Anspruch 12, **dadurch gekennzeichnet, daß** die Entfernung der Abprodukte mit einem Gas- bzw. Luftstrom erfolgt, der nicht auf das behandelte Gewebe gerichtet ist und dieses auch nicht oder nur in geringem Maße überstreicht, um so eine Austrocknung des Gewebes zu vermeiden.

## Claims

1. Arrangement for the extraction of waste products, such as smoke and tissue particles, in the ablation of biological tissue with the aid of laser radiation, wherein the laser radiation is directed onto the tissue through the orifice (3) of a tubular channel (2) and the waste products are sucked into the channel (2) through the orifice (3), **characterised in that** the inner wall of the channel has in the vicinity of the orifice (3) at least one outlet opening (8) for a gas stream which is directed to the centre of the channel (2) or is directed into the channel (2) in a direction substantially opposite to the radiation direction of the laser beam (4).

2. Arrangement as claimed in Claim 1, **characterised in that** the tubular channel (2) has a portion (13) which tapers conically towards the orifice (3).

3. Arrangement as claimed in Claim 2, **characterised in that** the tubular channel (2) has at least in the conically extending portion (13) two chambers (9, 10) which extend concentrically around its circumference, of which one chamber (10) is provided for delivering the gas to the outlet opening (8) and the second chamber (9) is provided for removing the extracted gas including the waste products.

4. Arrangement as claimed in any one of the preceding claims, **characterised in that** a plurality of outlet openings (8) are provided and are arranged in a radially symmetrical manner about the centre of the channel.

5. Arrangement as claimed in any one of Claims 1 to 3, **characterised in that** an outlet opening (8) is provided which runs round in an annular manner.

6. Arrangement as claimed in any one of the preceding claims, **characterised in that** the outlet openings are widened in the manner of diffusers.

7. Arrangement as claimed in any one of the preceding claims, **characterised in that** air is provided as gas and the outlet openings (8) are connected to a compressor or to a pressure vessel filled with air.

8. Arrangement as claimed in any one of the preceding claims, **characterised in that** the inner wall (6) of the channel (2) has a plurality of extraction openings which are disposed in a radially symmetrical manner about the centre of the channel and communicate with an extraction device.

9. Arrangement as claimed in any one of the preceding claims, **characterised in that**
- the total cross-section of the outlet openings (8) and the total cross section of the extraction openings (7),
- the overpressure at the outlet openings (8) and the vacuum at the extraction openings (7) and
- the flow velocities in the outlet openings (8) and the flow velocities in the extraction openings
are adapted to one another in such a way that the quantity of air drawn off per unit of time is greater than the quantity of air delivered through the outlet openings (8) by a factor from 1.1 to 1.3.

10. Arrangement as claimed in any one of the preceding claims, **characterised in that** a device is provided for alternately interrupting the laser radiation impinging on the tissue and the delivery and extraction of air.

11. Arrangement as claimed in any one of the preceding claims, **characterised in that** the channel is disposed so as to be pivotable relative to the laser radiation, wherein the laser radiation is surrounded by the channel (2) in a preferred orientation of the channel (2).

12. Method of operating an arrangement as claimed in Claims 1 to 11 for the extraction of waste products, such as smoke and tissue particles, in ablation with the aid of laser radiation, wherein the waste products which are produced during removal of material are removed from the treatment site in a phase in which the laser radiation is not simultaneously in active surgical use.

13. Method as claimed in Claim 12, **characterised in that** the removal of the waste products takes place with a gas or air stream which is not directed onto the treated tissue and which does not flow over the tissue or does so only to a slight extent, in order thus to avoid drying out of the tissue.

## Revendications

1. Système d'aspiration des déchets, tels que fumées et particules tissulaires, au cours de l'ablation d'un tissu biologique, au moyen d'un rayon laser, le rayon laser étant dirigé sur le tissu en passant à travers la bouche (3) d'un canal tubulaire (2) et les déchets étant aspirés à l'intérieur du canal (2) en passant par la bouche (3), **caractérisé en ce que** la paroi intérieure du canal à proximité de la bouche (3) comporte au moins un orifice de sortie (8) pour un flux de gaz qui est dirigé vers le milieu du canal (2) ou qui est dirigé à l'intérieur du canal (2) sensiblement en sens opposé à la direction de rayonnement du rayon laser (4).

2. Système selon la revendication 1, **caractérisé en ce que** le canal tubulaire (2) comporte une partie (13) qui se rétrécit en cône vers la bouche (3).

3. Système selon la revendication 2, **caractérisé en ce que** le canal tubulaire (2) comporte au moins dans la partie tronconique (13) deux chambres (9, 10) s'étendant concentriquement à sa périphérie, parmi lesquelles une chambre (10) est prévue pour l'acheminement du gaz vers l'orifice de sortie (8) et la deuxième chambre (9) pour l'évacuation du gaz aspiré, y compris les déchets.

4. Système selon l'une quelconque des revendications précédentes, **caractérisé en ce qu'**il est prévu plusieurs orifices de sortie (8) qui sont disposés en symétrie radiale autour du milieu du canal.

5. Système selon l'une quelconque des revendications 1 à 3, **caractérisé en ce qu'**il est prévu un orifice de sortie (8) périphérique annulaire.

6. Système selon l'une quelconque des revendications précédentes, **caractérisé en ce que** les orifices de sortie (8) sont évasés en forme de diffuseur.

7. Système selon l'une quelconque des revendications précédentes, **caractérisé en ce que** le gaz prévu est l'air et les orifices de sortie (8) sont reliés à un compresseur ou un récipient sous pression rempli d'air.

8. Système selon l'une quelconque des revendications précédentes, **caractérisé en ce que** la paroi intérieure (6) du canal (2) comporte plusieurs orifices d'aspiration (7), qui sont disposés en symétrie radiale autour du milieu du canal et qui sont reliés à un dispositif d'aspiration.

9. Système selon l'une quelconque des revendications précédentes, **caractérisé en ce que**
- la section totale des orifices de sortie (8) et la section totale des orifices d'aspiration (7),
- la surpression dans les orifices de sortie (8) et la dépression dans les orifices d'aspiration (7) et
- les vitesses d'écoulement dans les orifices de sortie (8) et les vitesses d'écoulement dans les orifices d'aspiration (7)
sont adaptées les unes aux autres de telle sorte que la quantité d'air aspirée par unité de temps est 1,1 à 1,3 fois supérieure à la quantité d'air conduite à travers les orifices de sortie (8).

10. Système selon l'une quelconque des revendications précédentes, **caractérisé en ce qu'**il est prévu un dispositif destiné à interrompre de manière alternée le rayonnement laser parvenant sur le tissu et la conduite et l'aspiration de l'air.

11. Système selon l'une quelconque des revendications précédentes, **caractérisé en ce que** le canal est agencé de manière pivotante par rapport au rayon laser, le rayon laser étant entouré par le canal (2) lorsque le canal (2) est dans une orientation préférée.

12. Procédé destiné à l'utilisation d'un système selon les revendications 1 à 11, destiné à l'aspiration de déchets, tels que des fumées et des particules tissulaires, dans lequel les déchets générés pendant l'ablation sont éliminés du site de traitement dans une phase dans laquelle le rayon laser n'est pas utilisé simultanément pour l'action chirurgicale.

13. Procédé selon la revendication 12, **caractérisé en ce que** l'enlèvement des déchets est effectué au moyen d'un flux de gaz ou d'air qui n'est pas dirigé sur le tissu traité et qui ne balaye pas ledit tissu ou le balaye seulement très faiblement, afin d'empêcher le dessèchement du tissu.
